# EUROPEAN PATENT SPECIFICATION

(11) **EP 3 755 207 B1**
(45) Date of publication and mention of the grant of the patent: **22.10.2025**
(21) Application number: 19703965.4
(22) Date of filing: 30.01.2019
(51) Int. Cl.: A61B 5/00, A61B 5/361

(54) **METHOD AND DEVICE FOR ARRHYTHMIA DETECTION**
VERFAHREN UND VORRICHTUNG ZUR ARRHYTHMEERKENNUNG
PROCÉDÉ ET DISPOSITIF DE DÉTECTION DE L'ARYTHMIE

(30) Priority: 23.02.2018 US 201862634215 P
(43) Date of publication of application: 30.12.2020
(73) Proprietor: BIOTRONIK SE & Co. KG, 12359 Berlin (DE)
(72) Inventor: HUEGERICH, Burkhard, Portland, Oregon 97202 (US); GARNER, Garth, Tigard, Oregon 97224 (US); WHITTINGTON, R. Hollis, Portland, Oregon 97202 (US)
(74) Representative: Biotronik Corporate Services SE
(86) International application number: PCT/EP2019/052248
(87) International publication number: WO 2019/162053

(56) References cited:
- EP-A1- 2 166 938
- EP-A1- 2 915 479
- EP-B1- 2 166 938
- WO-A1-2016/025704
- US-A1- 2010 099 996
- US-A1- 2011 196 247

## Description

The present disclosure relates to a method and a device for arrhythmia detection.

Cardiac monitors are medical devices that monitor the electrical activity of the heart by using electrodes in contact with the body tissue. The electrodes are usually located at the case of the medical device that may be implanted under the skin or be arranged in contact with the surface of the chest close in the vicinity of the heart. Alternatively, the electrodes are located at the distal end of at least one subcutaneous implanted electrode line connected to an implantable cardiac monitor. Implantable cardiac stimulators like pacemakers, defibrillators and cardiac resynchronization devices employ electrodes located at the distal end of electrode lines that are implanted in the heart chambers and connected to the implanted device and can provide also the functionality of an implantable cardiac monitor.

EP 2 166 938 B1 generally relates to implantable medical devices (IMDs), and, more particularly, relates to detecting noisy physiologic data intervals.

US 2010/0099996 A1 generally relate to implantable cardiac devices, including monitoring devices, pacemakers, defibrillators and cardioverters, which monitor, detect and classify cardiac events, for example atrial tachyarrhythmias. More particularly, embodiments relate to a method and device for detecting Atrial Fibrillation or Atrial Flutter by evaluating ventricular signals. Further, embodiments relate to methods for monitoring atrial events for use in implantable devices without atrial electrodes.

Cardiac events are detected by analyzing the electrical activity of the heart, e.g. by detecting arrhythmia. Noise events are identified by analyzing the electrical signal detected by the employed electrodes used to observe the electrical activity of the heart. Generally, noise events can be distinguished from electrical heart signals of interest because of their very different signature. Yet, due to the random nature of noise, the identification of noise cannot be performed with a 100% certainty.

False arrhythmia detections can occur during periods of high patient activity or active muscle noise. Currently employed noise and low signal management during arrhythmia detection does not achieve the desired PPV (Positive Predictive Value) in the presence of noise and/or low amplitude signals. Current algorithms remove or ignore single intervals and counts from arrhythmia detection buffers when a noise event is observed. The current algorithms are not aggressive enough to remove arrhythmia detections due to noise/interference.

Such false arrhythmia detections are not desired; therefore, the present invention addresses this shortcoming.

It is an object to provide improved technologies for arrhythmia detection. In particular, handling of noise shall be improved.

A method for arrhythmia detection within a heart signal of a body according to claim 1 and a device according to claim 14 are provided. Further embodiments are subject matter of dependent claims.

In one aspect, a method for arrhythmia detection within a heart signal of a patient is disclosed. The method is executed by a processor and comprises steps of: providing an input signal which refers to the heart signal of the patient, wherein the input signal comprises cardiac events and noise events; determining the cardiac events from the input signal in an arrhythmia detection window; and evaluating the arrhythmia detection window by taking into account at least one noise event occurring after an end of the arrhythmia detection window. The cardiac events are related to cardiac activity of interest of the heart of the patient, and the noise events are not related to cardiac activity of interest of the heart.

In another aspect, a device for arrhythmia detection is provided. The device comprises a sensing element which is configured to determine a heart signal of a heart of a patient, and a processor. The processor is configured to provide an input signal which refers to the heart signal, wherein the input signal comprises cardiac events and noise events; determine cardiac events from the input signal in an arrhythmia detection window; and evaluate the cardiac events in the arrhythmia detection window by taking into account at least one noise event occurring after an end of the arrhythmia detection window.

Electrical heart signals comprise of different shapes throughout a regular heart beat interval. Sometimes a heart signal detection system may miss-classify a specific interval phase (e.g. p-waves and t-waves might be falsely classified as QRS complexes). The cardiac activity of interest (or the electrical heart signals of interest) may be the QRS complex, p-waves or t-waves. Throughout the document, the term "cardiac event" refers to the cardiac activity (electrical heart signal) of interest.

A noise event may be, for example, muscle related activity or respiration related activity but also cardiac activity which is not of interest. E.g. if the cardiac events (cardiac activity of interest) are QRS complexes, then p-waves and t-waves may be considered to be noise events (because they are not of interest in this case). If the cardiac events are p-waves, then QRS and t-waves could be noise events. Finally, if the cardiac events are t-waves, QRS and p-waves could also be noise events.

The method is applicable to different kinds of arrhythmia like for example HVR (high ventricular rate), bradycardia, asystole and atrial fibrillation. The term "arrhythmia detection window" may refer to the phase in an ongoing arrhythmia detection sequence where an arrhythmia onset occurred but a complete episode was not confirmed, yet.

The disclosure relates to a new concept for prevention of false arrhythmia detections that may occur during periods of high patient activity or active muscle noise. Periods of noise in an electrocardiogram (ECG) are episodic, with periods of high noise lasting sometimes several minutes at a time but with small breaks between noise periods. This creates a situation where the device may detect interference but classify it as "true" detections although these detections occur within or during a longer period of noise and are actually false. These undetected noise events can appear as QRS detections. It is therefore important to identify these noise episodes. The presence of noise that occurs outside a given arrhythmia detection window may be used according to an embodiment as a means to discard the arrhythmia based on the likelihood it is false.

The method considers the presence of noise that occurs outside the actual arrhythmia detection window. Traditionally, an arrhythmia detection window starts if no noise is currently present and the specific conditions for an arrhythmia are met - such as the presence of a ventricular rate above an HVR threshold. An embodiment of the present invention provides and utilizes a certain "grace period" without noise events before and/or after the actual arrhythmia detection window. Even if the specific conditions for an arrhythmia are not met, yet the algorithm may let noise event counts to be accumulated up to a certain limit. These noise event counts need to be decremented by QRS intervals without noise events in order to enable the start of an arrhythmia detection window. These noise free QRS intervals (which may be the cardiac events) might or might not meet the specific arrhythmia detection conditions. The only condition the intervals have to meet is to be without intervening noise events.

The same principle may apply to the end of an arrhythmia detection window. Noise events right after the end of a successful arrhythmia detection window can de-validate the positive detection results of the preceding arrhythmia detection window. The length of the grace period after the arrhythmia detection window may be determined by the accumulated noise counts before the arrhythmia detection window. Additionally, if noise events occur within an arrhythmia detection window, instead of merely cancel an ongoing arrhythmia detection window, the noise event counter may get pre-set with a certain count, preventing an immediate start of the next arrhythmia detection window. When there are high periods of noise, arrhythmia detection will be inhibited or prevented, and only when interference is absent will it be possible to detect arrhythmias. Noise that occurs in the period after detection may act as a further filter, causing the episode to be discarded when high noise occurs immediately afterwards. By identifying whether the episode occurs during an episode of noise that may precede or follow the arrhythmia detection, specificity can be increased significantly.

One advantage of the method according to an embodiment is that arrhythmia detection results are achieved with a higher PPV (positive predictive value) especially in the presence of noise and/or low amplitude signals. This allows for presentation of the detection results with a higher confidence to an interested physician. The ratio of correct to incorrect snapshots by removing confounding noisy snapshots will be significantly improved as well as customer perception and the clinical utility of the method.

The embodiments described here are motivated by the knowledge of the clinical results of the current arrhythmia detection algorithms and the behavior of noise during activities of daily living. Utilizing the noise properties, an embodiment of the present invention is provided that recognizes periods with higher likelihoods of false detections and prevents false detections of arrhythmias. The solution may be implemented in device firmware using currently available technology in the IPG platform (IPG - implantable pulse generator) without major structural changes, impact on system architecture or power consumption.

The step of determining the cardiac events from the input signal in the arrhythmia detection window may be terminated if a noise event is detected in the arrhythmia detection window.

The step of determining the cardiac events from the input signal in the arrhythmia detection window may be terminated if a predetermined number of cardiac events matching the arrhythmia criteria are detected in the arrhythmia detection window.

The arrhythmia detection window may be followed by a confirmation period after the end of the arrhythmia detection window. The arrhythmia detection window may be confirmed when no noise event is detected during the confirmation period. The arrhythmia detection window may be discarded when a noise event is detected during the confirmation period. In this way, noise right after the active arrhythmia detection window is taken into account as well which may further increase the PPV results.

The duration of the confirmation period may be determined by a predetermined number of cardiac events after the end of the arrhythmia detection window. The predetermined number of cardiac events after the end of the arrhythmia detection window may depend on the number of noise events occurring before the start of the arrhythmia detection window.

In one embodiment, the method may further comprise providing an event counter which is configured to count cardiac events and noise events. Before the start of the arrhythmia detection window, the event counter is increased by a first value for each detected cardiac event, and the event counter is decreased by a second value for each detected noise event. The arrhythmia detection window is started when the event counter is equal to or more than a first predetermined value. The first predetermined value may be 0. Such a value is easy to implement and simplifies calculations.

The second value may be twice as high as the first value. For example, the first value may be 1 and the second value may be 2. The rationale would be that noise (if not detected later on) can cause the registration of two false intervals of a higher rate which is countered by the relationship between increase and decrease of the event counter. Tests have shown good results for such ratio.

The event counter may be increased up to a maximum of a first predetermined limit. For example, the first predetermined limit may be 0. The event counter may be decreased down to a minimum of a second predetermined limit, the second predetermined limit may be -4. It is of advantage to limit the decrementation to a fixed value to avoid having a long sequence of noise inhibit the detection of a real arrhythmia soon after.

For example, noise events may decrement the event counter below 0. How far down noise events are allowed to decrement the event counter is limited. Assuming noise events decremented this event counter below 0 and if the first predetermined limit for the start of an arrhythmia detection window is set to 0 or higher, than regular events need to increment the event counter until it reaches that set limit to allow the start of an arrhythmia detection window.

The method may further comprise after starting the arrhythmia detection window: increasing the event counter by the first value for each detected cardiac event, and decreasing the event counter by a third value if a noise event is detected. The arrhythmia detection window can be terminated either (i) if the event counter is lower than the first predetermined value or (ii) if the event counter is equal to or more than a second predetermined value. The third value may be 16. The second predetermined value may be 8 or 16.

If the arrhythmia detection window is terminated because the event counter is lower than the first predetermined value, this results in failed arrhythmia detection. By decreasing the event counter by the third value, the event counter may turn negative which in turn has two consequences. First, the actual arrhythmia detection window is terminated (since the event counter is lower than the first predetermined value). Second, after termination the next arrhythmia detection window is not started directly but only when the event counter reaches the first predetermined value (e.g. zero).

In case the arrhythmia detection window is terminated because the event counter is equal to or more than the second predetermined value, this results in successful arrhythmia detection. In this case, a suitable number of consecutive cardiac events indicating the presence of an arrhythmia have been recorded without interrupting noise events.

Also, the arrhythmia detection window may be terminated if the cardiac events stop indicating the presence of an arrhythmia within the arrhythmia detection window. This would result in failed arrhythmia detection.

If successful arrhythmia detection was established at the end of the arrhythmia detection window, a termination period may be started by setting the event counter to a fourth value.

During the termination period, the event counter is decreased with every cardiac event that does not indicate the presence of an arrhythmia. The arrhythmia detection sequence is terminated as soon as the event counter is equal to or less than the first predetermined value. The fourth value may be 5. The arrhythmia detection sequence comprises the arrhythmia detection window as well as evaluating noise events before and/or after the arrhythmia detection window.

**In** another embodiment, the method may further comprise providing a first event counter which is configured to count cardiac events, and providing a second event counter which is configured to count noise events. Before the start of the arrhythmia detection window, the second event counter is increased by a fifth value for each detected noise event and the second event counter is decreased by a sixth value for each detected cardiac event. The arrhythmia detection window is started when the second event counter is equal to or less than a third predetermined value. The first event counter is started when the arrhythmia detection window is started. The third predetermined value may be 0.

The fifth value may be twice the sixth value. For example, the fifth value may be 2 and the sixth value may be 1.

The second event counter may also be called noise counter. The second event counter may be increased up to a maximum of a third predetermined limit. The third predetermined limit may be 8. The second event counter may be decreased down to a minimum of a fourth predetermined limit. The fourth predetermined limit may be 0.

The method may further comprise after starting the arrhythmia detection window: increasing the second event counter by the fifth value for each detected noise event, and increasing the first event counter by a seventh value for each detected cardiac event. The arrhythmia detection window may be terminated either (i) if the second event counter is more than the third predetermined value or (ii) if the first event counter is equal to or more than a fourth predetermined value. The fourth predetermined value may be 8 or 16.

If the arrhythmia detection window is terminated because the second event counter is more than the third predetermined value, this results in failed arrhythmia detection.

If the arrhythmia detection window is terminated because the first event counter is equal to or more than the fourth predetermined value, this results in successful arrhythmia detection.

Also, the arrhythmia detection window may be terminated if the cardiac events stop indicating the presence of an arrhythmia, resulting in failed arrhythmia detection.

After the arrhythmia detection window is terminated because the first event counter is equal to or more than the fourth predetermined value, a confirmation period may be started by setting the second event counter (noise counter) to an eighth value. During the confirmation period the second event counter is decreased by one with every cardiac event (without noise) and successful arrhythmia detection result is confirmed as soon as the second event counter is equal to or less than zero. The eighth value may be obtained by doubling the present value of the second event counter plus adding an offset value. The offset value may be 2. If a noise event occurs during the confirmation period this indicates failed arrhythmia detection.

The method may further comprise starting a termination period, by setting the first event counter to a ninth value; and, during termination period, decreasing the first event counter by one with every cardiac event that does not indicate the presence of an arrhythmia and terminate the arrhythmia detection sequence as soon as the first event counter is equal to or less than the third predetermined value. The ninth value may be 5.

The features disclosed with respect to the method can also be applied to the device and vice versa.

In the following, exemplary embodiments are described.
- Fig. 1: shows a schematic representation of a cardiac device,
- Fig. 2a: shows a diagram of a first scenario according to a first embodiment,
- Fig. 2b: shows a diagram of a second scenario according to the first embodiment,
- Fig. 3: shows a diagram of a first scenario according to a second embodiment,
- Fig. 4: shows a diagram of a second scenario according to the second embodiment,
- Fig. 5: shows a diagram of a third scenario according to the second embodiment.

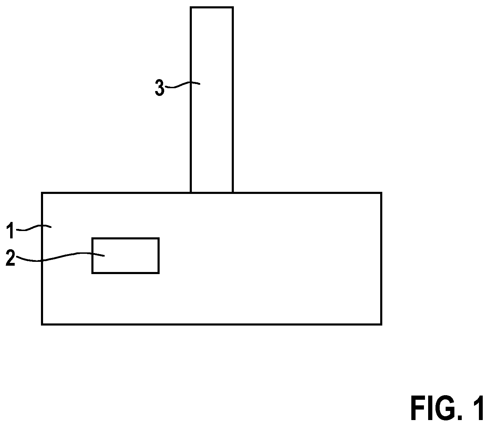

Figure 1 shows an exemplary embodiment of a cardiac device 1. In this embodiment, the cardiac device 1 is an implantable cardiac monitor and comprises a sensing element 3, for example a sensing electrode. A processor 2 is arranged within a housing of the cardiac device 1. The cardiac device 1 may comprise further sensing electrodes. In one embodiment, the cardiac device 1 comprises three sensing electrodes (not shown). The cardiac device 1 may include three input channels that receive different projections of the mean heart signal as input signals. Each input channel may use a single pair of the three sensing electrodes and each input channel may sense a different planar projection of the mean heart signal, wherein the projections are coincident in time. Noise on each input channel may be determined, in part, by the local activity around the sensing electrodes in that particular pair. Combining the three input channels into a single input channel may increase the signal to noise ratio by smoothing some of the random noise associated with each input channel, while emphasizing the QRS-signals or QRS complexes.

The following discussion refers to a single input channel and a single signal, respectively as well as to a combined input channel and a combined signal, respectively. The present disclosure relates to single and combined signals.

The cardiac device 1 monitors the electrocardiogram (ECG) for QRS events, arrhythmia events and noise events. Detected QRS events and arrhythmia events are marked by a sense marker and noise events are marked by a noise marker. The markers are part of an input signal to the method. In the following Figures, such input signals including sense markers and noise markers are depicted.

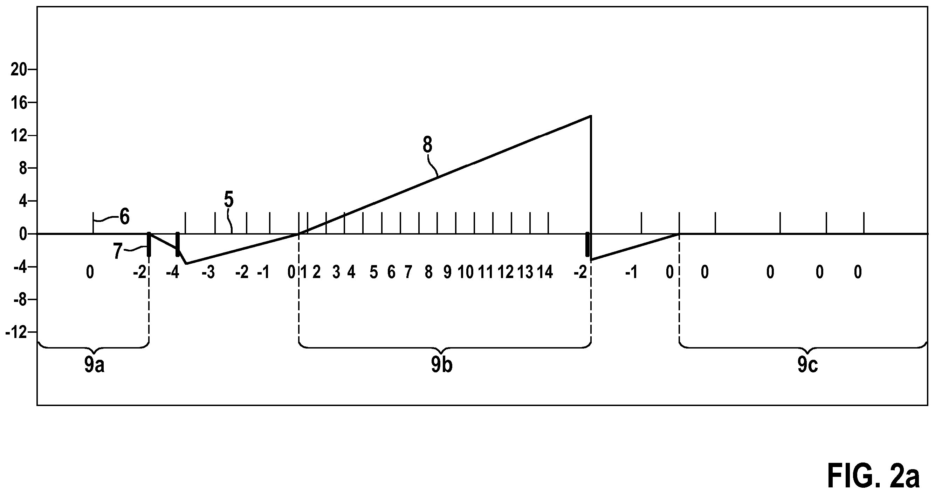

A first scenario of a first embodiment is shown in Figure 2a. Figure 2a shows a timeline from an input signal 5 including the related sense markers or cardiac events (also called noise-free events) 6 and noise markers or noise events 7. Further depicted is an event counter 8 which counts cardiac events 6 and noise events 7. Each noise event 7 decreases or decrements the event counter 8 and each cardiac event 6 increases or increments the event counter 8. A scale for the value of the event counter 8 is given at the left side of the diagram.

Further depicted in Figure 2a are arrhythmia detection windows 9. During the arrhythmia detection windows 9 the actual detection of an arrhythmia takes place. While the method concerns arrhythmia detection it can be seen as focusing on defining or creating an arrhythmia detection window 9. Here, three arrhythmia detection windows 9a, 9b and 9c are shown. The left arrhythmia detection window 9a can be seen as a preceding or lapsed arrhythmia detection window. The middle arrhythmia detection window 9b can be seen as a current or active arrhythmia detection window. The right arrhythmia detection window 9c can be seen as a subsequent arrhythmia detection window.

The following rules may be implemented in the method. In addition to noise conditions within an active arrhythmia detection window 9b, noise conditions or noise events 7 right before the start of the active arrhythmia detection window 9b are monitored as well.

If a noise event 7 is detected and the event counter 8 is larger than 0 which means that there is an active arrhythmia detection window 9b, instead of decrementing or setting the event counter 8 to 0, a fixed value, in this example of 16, is subtracted. Therefore, the event counter 8 can result in a negative value. The rational for this modification is that noise detection within the active arrhythmia detection window 9b most likely hints towards more and probably undetected noise and needs to be counter-acted aggressively.

If a noise event 7 is detected and the event counter 8 is equal to or less than 0, the event counter 8 is decremented by a programmable value, in this example 2. Decrementing the event counter 8 is limited to a fixed negative limit.

If no noise event 7 is detected and the interval's rate is below the HVR threshold and event counter 8 is less than 0, the event counter 8 is incremented by a programmable value, in this example 1.

If no noise event 7 is detected and the interval's rate is above the HVR threshold, the event counter 8 is incremented by 1.

In the following, the function of the method according to an embodiment is explained with regard to the curve of the event counter 8, i.e. at the end of the window preceding arrhythmia detection 9a. The starting point for this discussion is the first noise event 7 at which the event counter 8 is decremented by two from the value of zero to the value of minus two. The next noise event decrements the event counter 8 further to minus four. The next four cardiac events 6 which may include QRS and arrhythmia events each increment the event counter 8 by one which results in a value of zero for the event counter 8.

Having reached the predetermined limit of zero for the event counter 8, the active arrhythmia detection window 9b starts. During the active arrhythmia detection window 9b the event counter 8 is incremented by cardiac events 6 to a value of fourteen. Then, a noise event 7 is detected inside the active arrhythmia detection window 9b which leads to a decrease of the event counter 8 by a third value, in this example of sixteen. The event counter 8 is thus reduced to the negative value of minus 2. As the event counter 8 is below the predetermined limit of zero the active arrhythmia detection window 9b is terminated and discarded as it includes noise. Two cardiac events 6 (without noise) are needed to start the subsequent arrhythmia detection window 9c as the event counter 8 starts with a value of minus two after the active arrhythmia detection window 9b.

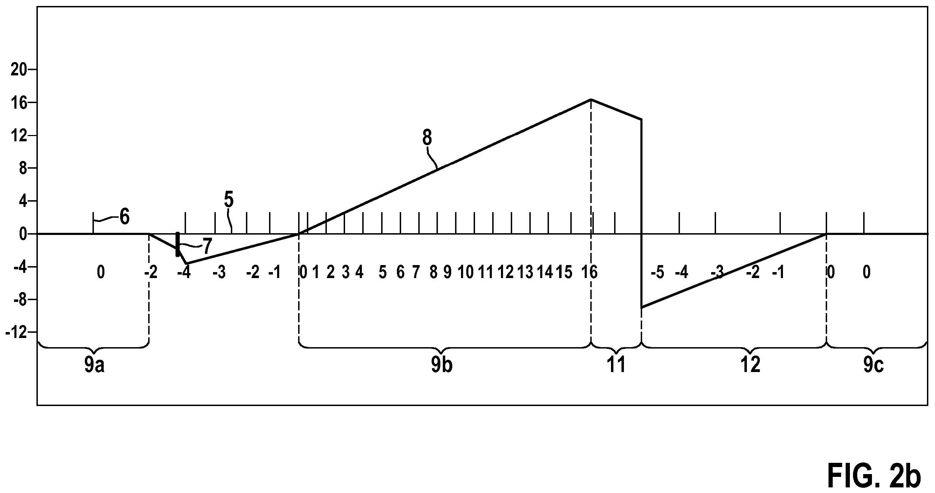

Figure 2b shows a second scenario of the first embodiment. The beginning is similar to the situation shown in Figure 2a. After the end of the preceding arrhythmia detection window 9a, the event counter 8 has a value of 0. Two successive noise events 7 lead to a reduction of the event counter 8 to a value of -4. Following, four cardiac events 6 are registered, each cardiac event 6 increasing the event counter 8 by a value of 1 which results in a value of 0 for the event counter 8. Having reached the predetermined limit of 0 for the event counter 8, the active arrhythmia detection window 9b starts. During the active arrhythmia detection window 9b the event counter 8 is incremented by cardiac events 6 until it reaches a predetermined value (here 16, other values are possible).

With the termination of the active arrhythmia detection window 9b because the predetermined number of cardiac events 6 (with arrhythmia indication) has been reached, a confirmation period 11 / confirmation state 11 starts. The length or duration of the confirmation state 11 is set to a value of 2 (other values are possible). The event counter 8 decrements during the confirmation state 11 by one for each detected cardiac event 6. After two cardiac events 6 (without noise), the confirmation state 11 is ended successfully.

At the end of the confirmation state 11 the event counter 8 is set to a predetermined value, in this example to a value of -5. A termination period 12 / termination state 12 follows the confirmation state 11. During the termination state 12 the event counter 8 is incremented for each cardiac event 6 (without arrhythmia indication) by the value of 1. The termination state 12 ends when the event counter 8 reaches the value of 0. Then, the next arrhythmia detection window 9c commences. In the example of Figure 2b, the detection window 9b was successful as no noise event 7 was detected inside the confirmation state 11.

If a noise event 7 would have been detected in the confirmation state 11, the active arrhythmia detection window 9b would be discarded.

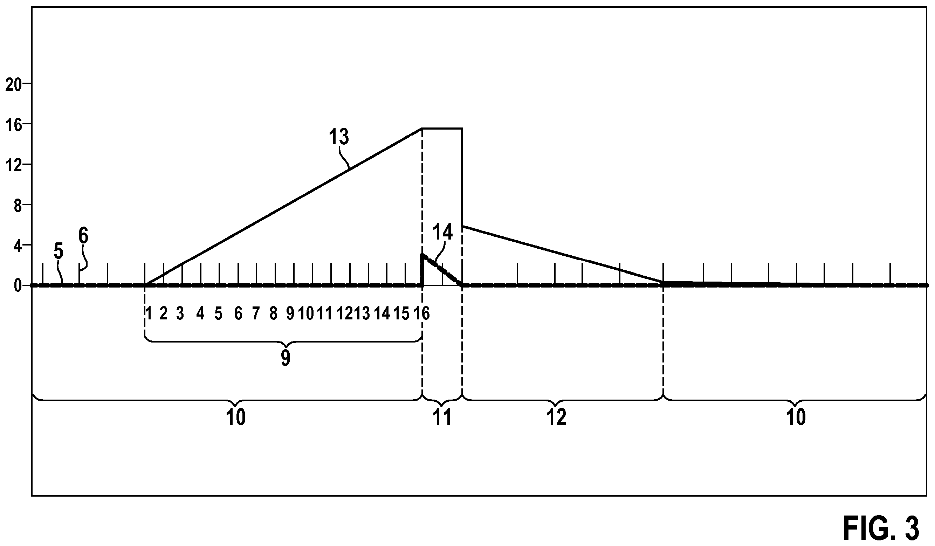

Figures 3 to 5 represent a second embodiment of an implementation of the method by utilizing a first event counter 13 and a second event counter 14 (also called noise counter). Instead of allowing negative event counter values in case of noise events as done in the first embodiment, the noise counter now counts the noise events. This approach supports the extension of the algorithm towards considering noise events after the end of the active arrhythmia detection window.

The method according to the second embodiment (as shown in Figures 3 to 5) is extended with regard as shown in Figure 2a in the way that noise events right after the active arrhythmia detection window are taken into account as well. Depending on the existence of noise events right before the start of an active arrhythmia detection window, the time will vary for how long the algorithm looks for noise events right after the active arrhythmia detection window.

Different states can be defined, namely a detection state, a confirmation state / confirmation period, and a termination state / termination period. The detection state replicates the behavior of Figure 2a. In other words, Figure 2a shows detection states only since confirmation and termination states do not show up due to the circumstances of the situation (noise during active detection terminates immediately the current detection sequence and starts a new detection sequence).

The confirmation state extends the behavior of the algorithm. At the end of an active arrhythmia detection window a time window or confirmation period is used to identify noise events. If noise events are detected within this time window, the current detection is classified as failed and is aborted. The next state will be a new detection state. On the other side, if no noise event is detected, the detection is classified as confirmed or successful and the next state will be the termination state.

The termination state is started with the end of a successful confirmation state. The end or termination of the termination state is decided as known, i.e. a predefined number of intervals with rates below the HVR rate threshold are detected. Then, a new detection state is entered again.

Figures 3 to 5 depict behavior for different noise scenarios.

Figure 3 shows a diagram with no noise events. The situation shown in Figure 3 demonstrates that in the absence of noise events at the beginning of the active arrhythmia detection window 9, only a short period of intervals (2 intervals in the above case) is used to confirm the arrhythmia detection.

A detection state 10 ends with the termination of the active detection window 9 because the predetermined number of cardiac events 6 (with arrhythmia indication) has been reached. Directly after the detection state 10, a confirmation state 11 starts. The length or duration of the confirmation state 11 is determined by the noise counter 14. The noise counter 14 is not amended in this example since no noise was encountered before the start of the active detection window. Nevertheless, an offset value of two is added to the noise counter 14 at the end of the detection window 9 or the end of the detection state 10. For each cardiac event (without noise) the noise counter 14 is decremented by one. As the noise counter 14 reaches zero, the confirmation state 11 is ended successfully. During the confirmation state 11 the first event counter 13 is not altered.

At the end of the confirmation state 11 the first event counter 13 is set to a predetermined value, in this example to a value of five. A termination state 12 follows the confirmation state 11. During the termination state 12 the first event counter 13 is decremented for each cardiac event 6 (without arrhythmia indication) by the value of one. The termination state 12 ends when the first event counter 13 reaches zero. Then, the next detection state 10 commences. In the example of Figure 3, the detection window 9 was successful as no noise event was detected inside the confirmation state 11.

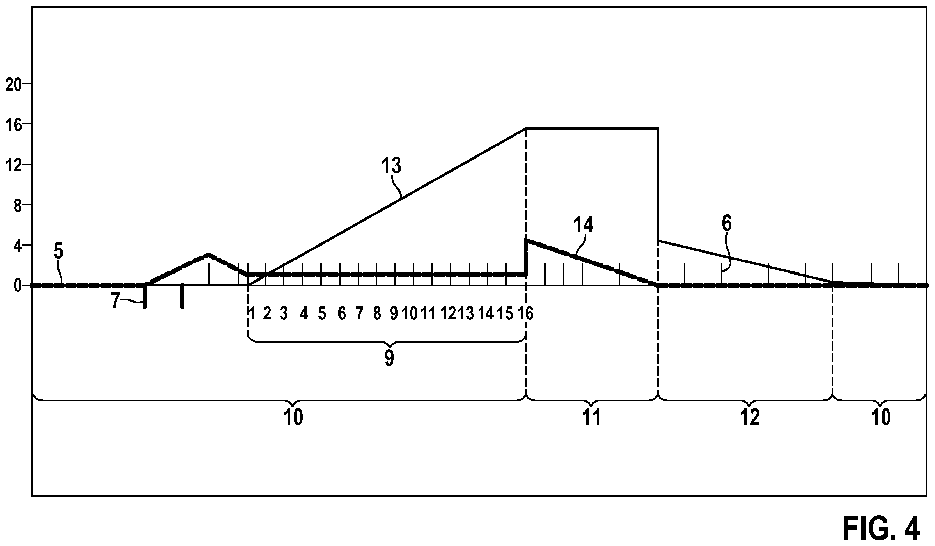

Figure 4 shows a diagram with noise events 7 before the start and a successful confirmation. Figure 4 shows noise events 7 before the start of an active arrhythmia detection window 9, inhibiting the start of the arrhythmia detection window 9. The noise counter 14 is incremented in the example by a value of two for each noise event and decremented by a value of one for each cardiac event 6. As soon as the noise counter is decremented below the value of 2, the active arrhythmia detection window 9 starts because of intervals having rates higher than the HVR rate threshold. Once the first event counter 13 reaches the event counter limit of sixteen, the confirmation state 11 starts. Because of the initial noise events 7 at the start of the active arrhythmia detection window 9, the confirmation state 11 is elongated to five intervals. This is due to the noise counter remaining value of one at the start of the actual detection window. Because of that an offset value of 4 is added to the noise counter 14 at the end of the arrhythmia detection window 9 or the end of the detection state 10. As soon as the confirmation state 11 for this episode is established, the regular termination state 12 takes over.

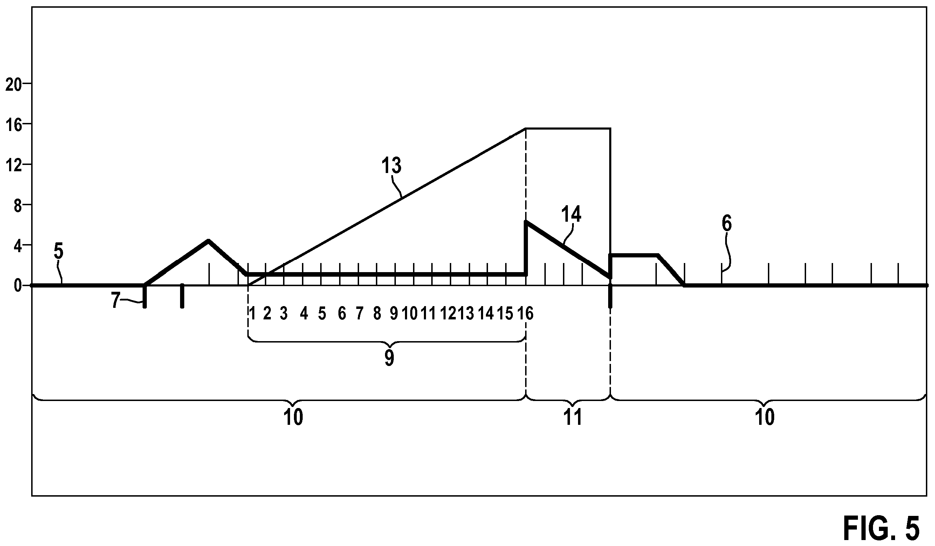

Figure 5 shows a diagram with noise events 7 before the start and a failed confirmation. As in Figure 4, Figure 5 shows noise events 7 before the start of an active arrhythmia detection window 9, inhibiting the start of the arrhythmia detection window 9. After that, in the absence of noise events 7, the active arrhythmia detection window 9 starts because of intervals having rates higher than the HVR rate threshold. Once the first event counter 13 reaches the event counter limit of sixteen, the confirmation state 11 starts. Because of the initial noise events 7 at the start of the active arrhythmia detection window 9, the confirmation state 11 is elongated to five intervals. This is due to the noise counter remaining value of one at the start of the actual detection window. Because of that an offset value of 4 is added to the noise counter 14 at the end of the arrythmia detection window 9 or the end of the detection state 10. Later, still within the confirmation state 11, a noise event 7 causes a fail of the confirmation for this episode. Now, the next regular detection state 10 starts with the noise counter value set to two.

## Claims

1. A method for arrhythmia detection within a heart signal of a patient, wherein the method is executed by a processor (2) and comprises steps of:
- providing an input signal (5) which refers to the heart signal of the patient, wherein the input signal (5) comprises cardiac events (6) and noise events (7), wherein the cardiac events (6) are related to cardiac activity of interest of the heart of the patient, and wherein the noise events (7) are not related to cardiac activity of interest of the heart;
- determining the cardiac events (6) from the input signal (5) in an arrhythmia detection window (9);
- evaluating the arrhythmia detection window (9) by taking into account at least one noise event (7) occurring after an end of the arrhythmia detection window (9).

2. The method of claim 1, wherein the step of determining the cardiac events (6) from the input signal (5) in the arrhythmia detection window (9) is terminated if a noise event (7) is detected in the arrhythmia detection window (9).

3. The method of claim 1 or 2, wherein the step of determining the cardiac events (6) from the input signal (5) in the arrhythmia detection window (9) is terminated if a predetermined number of cardiac events (6) is detected in the arrhythmia detection window (9).

4. The method of any one of the preceding claims, wherein the arrhythmia detection window (9) is followed by a confirmation period (11) after the end of the arrhythmia detection window (9), wherein the arrhythmia detection window (9) is confirmed when no noise event (7) is detected during the confirmation period (11), and wherein the arrhythmia detection window (9) is discarded when a noise event (7) is detected during the confirmation period (11).

5. The method of claim 4, wherein duration of the confirmation period (11) is determined by a predetermined number of cardiac events (6) after the end of the arrhythmia detection window (9), and wherein the predetermined number of cardiac events (6) after the end of the arrhythmia detection window (9) depends on the number of noise events (7) occurring before the start of the arrhythmia detection window (9).

6. The method of any one of the preceding claims, further comprising:
- providing an event counter (8) which is configured to count cardiac events (6) and noise events (7),
- before the start of the arrhythmia detection window (9):
- increasing the event counter (8) by a first value for each detected cardiac event (6);
- decreasing the event counter (8) by a second value for each detected noise event (7),
- starting the arrhythmia detection window (9) when the event counter (8) is equal to or more than a first predetermined value.

7. The method of claim 6, wherein the second value is twice as high as the first value.

8. The method of claim 6 or 7, further comprising
- after starting the arrhythmia detection window (9),
- increasing the event counter (8) by the first value for each detected cardiac event (6);
- decreasing the event counter (8) by a third value if a noise event (7) is detected,
- terminating the arrhythmia detection window (9) either
- if the event counter (8) is lower than the first predetermined value or
- if the event counter (8) is equal to or more than a second predetermined value.

9. The method of claim 8, wherein after the arrhythmia detection window (9) is terminated because the event counter (8) is equal to or more than the second predetermined value, a termination period (12) is started by setting the event counter (8) to a fourth value, wherein during the termination period (12), the event counter (8) is decreased with every cardiac event (6) that does not indicate the presence of an arrhythmia, and wherein an arrhythmia detection sequence is terminated as soon as the event counter (8) is equal to or less than the first predetermined value.

10. The method of any one of claims 1 to 5, further comprising:
- providing a first event counter (13) which is configured to count cardiac events (6),
- providing a second event counter (14) which is configured to count noise events (7),
- before the start of the arrhythmia detection window (9):
- increasing the second event counter (14) by a fifth value for each detected noise event (7);
- decreasing the second event counter (14) by a sixth value for each detected cardiac event (6),
- starting the arrhythmia detection window (9) when the second event counter (14) is equal to or less than a third predetermined value,
wherein the first event counter (13) is started when the arrhythmia detection window (9) is started.

11. The method of claim 10, further comprising
- after starting the arrhythmia detection window (9),
- increasing the first event counter (13) by a seventh value for each detected cardiac event (6);
- increasing the second event counter (14) by the fifth value for each detected noise event (7),
- terminating the arrhythmia detection window (9) either
- if the first event counter (13) is equal to or more than a fourth predetermined value or
- if the second event counter (14) is more than the third predetermined value.

12. The method of claim 11, wherein after the arrhythmia detection window (9) is terminated because the first event counter (13) is equal to or more than the fourth predetermined value, a confirmation period (11) is started by setting the second event counter (14) to an eighth value, and during the confirmation period (11) the second event counter (14) is decreased by one with every cardiac event (6) and successful arrhythmia detection result is confirmed as soon as the second event counter (14) is equal to or less than zero.

13. The method of claim 12, further comprising
- starting a termination period (12), by setting the first event counter (13) to a ninth value;
- during termination period (12), decreasing the first event counter (13) by one with every cardiac event (6) that does not indicate the presence of an arrhythmia and terminate the arrhythmia detection sequence as soon as the first event counter (13) is equal to or less than the third predetermined value.

14. A device (1) for arrhythmia detection comprising
- a sensing element (3) which is configured to determine a heart signal of a heart of a patient, and
- a processor (2) which is configured to
- provide an input signal (5) which refers to the heart signal, wherein the input signal (5) comprises cardiac events (6) and noise events (7), wherein the cardiac events (6) are related to cardiac activity of interest of the heart of the patient, and wherein the noise events (7) are not related to cardiac activity of interest of the heart;
- determine cardiac events (6) from the input signal (5) in an arrhythmia detection window (9), and
- evaluate the cardiac events (6) in the arrhythmia detection window (9) by taking into account at least one noise event (7) occurring after an end of the arrhythmia detection window (9).

## Patentansprüche

1. Verfahren zur Arrhythmie-Erfassung in einem Herzsignal eines Patienten, wobei das Verfahren von einem Prozessor (2) ausgeführt wird und folgende Schritte umfasst:
- Bereitstellen eines Eingangssignals (5), das sich auf das Herzsignal des Patienten bezieht, wobei das Eingangssignal (5) kardiale Ereignisse (6) und Rauschereignisse (7) umfasst, wobei die kardialen Ereignisse (6) mit der interessierenden Herzaktivität des Herzens des Patienten in Beziehung stehen, und wobei die Rauschereignisse (7) nicht mit der interessierenden Herzaktivität des Herzens in Beziehung stehen;
- Bestimmen der kardialen Ereignisse (6) aus dem Eingangssignal (5) in einem Arrhythmie-Erfassungsfenster (9);
- Auswerten des Arrhythmie-Erfassungsfensters (9) unter Berücksichtigung mindestens eines Rauschereignisses (7), das nach einem Ende des Arrhythmie-Erfassungsfensters (9) auftritt.

2. Verfahren nach Anspruch 1, wobei der Schritt des Bestimmens der kardialen Ereignisse (6) aus dem Eingangssignal (5) im Arrhythmie-Erfassungsfenster (9) beendet wird, wenn ein Rauschereignis (7) im Arrhythmie-Erfassungsfenster (9) erfasst wird.

3. Verfahren nach Anspruch 1 oder 2, wobei der Schritt des Bestimmens der kardialen Ereignisse (6) aus dem Eingangssignal (5) im Arrhythmie-Erfassungsfenster (9) beendet wird, wenn eine vorbestimmte Anzahl von kardialen Ereignissen (6) im Arrhythmie-Erfassungsfenster (9) erfasst wird.

4. Verfahren nach einem der vorhergehenden Ansprüche, wobei auf das Arrhythmie-Erfassungsfenster (9) eine Bestätigungsperiode (11) nach dem Ende des Arrhythmie-Erfassungsfensters (9) folgt, wobei das Arrhythmie-Erfassungsfenster (9) bestätigt wird, wenn während der Bestätigungsperiode (11) kein Rauschereignis (7) erfasst wird, und wobei das Arrhythmie-Erfassungsfenster (9) verworfen wird, wenn während der Bestätigungsperiode (11) ein Rauschereignis (7) erfasst wird.

5. Verfahren nach Anspruch 4, wobei die Dauer der Bestätigungsperiode (11) durch eine vorbestimmte Anzahl von kardialen Ereignissen (6) nach dem Ende des Arrhythmie-Erfassungsfensters (9) bestimmt wird, und wobei die vorbestimmte Anzahl von kardialen Ereignissen (6) nach dem Ende des Arrhythmie-Erfassungsfensters (9) von der Anzahl von Rauschereignissen (7) abhängt, die vor dem Start des Arrhythmie-Erfassungsfensters (9) auftreten.

6. Verfahren nach einem der vorangegangenen Ansprüche umfasst ferner:
- Bereitstellen eines Ereigniszählers (8), der so konfiguriert ist, dass er kardiale Ereignisse (6) und Rauschereignisse (7) zählt,
- vor dem Start des Arrhythmie-Erfassungsfensters (9):
- Erhöhen des Ereigniszählers (8) um einen ersten Wert für jedes erfasste kardiale Ereignis (6);
- Verringern des Ereigniszählers (8) um einen zweiten Wert für jedes erfasste Rauschereignis (7),
- Starten des Arrhythmie-Erfassungsfensters (9), wenn der Ereigniszähler (8) gleich oder größer als ein erster vorbestimmter Wert ist.

7. Verfahren nach Anspruch 6, wobei der zweite Wert doppelt so hoch wie der erste Wert ist.

8. Verfahren nach Anspruch 6 oder 7 umfasst ferner
- nach dem Starten des Arrhythmie-Erfassungsfensters (9),
- Erhöhen des Ereigniszählers (8) um den ersten Wert für jedes erfasste kardiale Ereignis (6);
- Verringern des Ereigniszählers (8) um einen dritten Wert, wenn ein Rauschereignis (7) erfasst wird,
- Beenden des Arrhythmie-Erfassungsfensters (9) entweder
- wenn der Ereigniszähler (8) niedriger ist als der erste vorbestimmte Wert oder
- wenn der Ereigniszähler (8) gleich oder größer als ein zweiter vorbestimmter Wert ist.

9. Verfahren nach Anspruch 8, wobei nachdem das Arrhythmie-Erfassungsfensters (9) beendet ist/wurde, weil der Ereigniszähler (8) gleich oder größer als der zweite vorbestimmte Wert ist, eine Beendigungsperiode (12) durch Setzen des Ereigniszählers (8) auf einen vierten Wert gestartet wird, wobei während der Beendigungsperiode (12), der Ereigniszähler (8) bei jedem kardialen Ereignis (6), das nicht das Vorhandensein einer Arrhythmie anzeigt, verringert wird, und wobei eine Arrhythmie-Erfassungssequenz beendet wird, sobald der Ereigniszähler (8) gleich oder kleiner als der erste vorbestimmte Wert ist.

10. Verfahren nach einem der Ansprüche 1 bis 5 umfasst ferner:
- Bereitstellen eines ersten Ereigniszählers (13), der so konfiguriert ist, dass er kardiale Ereignisse (6) zählt,
- Bereitstellen eines zweiten Ereigniszählers (14), der so konfiguriert ist, dass er Rauschereignisse (7) zählt,
- vor dem Start des Arrhythmie-Erfassungsfensters (9):
- Erhöhen des zweiten Ereigniszählers (14) um einen fünften Wert für jedes erfasste Rauschereignis (7);
- Verringern des zweiten Ereigniszählers (14) um einen sechsten Wert für jedes erfasste kardiale Ereignis (6),
- Starten des Arrhythmie-Erfassungsfensters (9), wenn der zweite Ereigniszähler (14) gleich oder kleiner als ein dritter vorbestimmter Wert ist,
wobei der erste Ereigniszähler (13) gestartet wird, wenn das Arrhythmie-Erfassungsfenster (9) gestartet wird.

11. Verfahren nach Anspruch 10 umfasst ferner
- nach dem Start des Arrhythmie-Erfassungsfensters (9),
- Erhöhen des ersten Ereigniszählers (13) um einen siebten Wert für jedes erfasste kardiale Ereignis (6);
- Erhöhen des zweiten Ereigniszählers (14) um den fünften Wert für jedes erfasste Rauschereignis (7),
- Beenden des Arrhythmie-Erfassungsfensters (9) entweder
- wenn der erste Ereigniszähler (13) gleich oder größer als ein vierter vorbestimmter Wert ist oder
- wenn der zweite Ereigniszähler (14) größer als der dritte vorbestimmte Wert ist.

12. Verfahren nach Anspruch 11, wobei nachdem das Arrhythmie-Erfassungsfensters (9) beendet ist/wurde, weil der erste Ereigniszähler (13) gleich oder größer als der vierte vorbestimmte Wert ist, eine Bestätigungsperiode (11) gestartet wird, indem der zweite Ereigniszähler (14) auf einen achten Wert gesetzt wird, und während der Bestätigungsperiode (11) der zweite Ereigniszähler (14) mit jedem kardialen Ereignis (6) um eins verringert wird und ein erfolgreiches Arrhythmie-Erfassungsergebnis bestätigt wird, sobald der zweite Ereigniszähler (14) gleich oder kleiner als null ist.

13. Verfahren nach Anspruch 12 umfasst ferner
- Starten einer Beendigungsperiode (12), indem der erste Ereigniszähler (13) auf einen neunten Wert gesetzt wird;
- während der Beendigungsperiode (12) den ersten Ereigniszähler (13) bei jedem kardialen Ereignis (6), das nicht das Vorhandensein einer Arrhythmie anzeigt, um eins zu verringern und die Arrhythmie-Erfassungssequenz zu beenden, sobald der erste Ereigniszähler (13) gleich oder kleiner als der dritte vorbestimmte Wert ist.

14. Vorrichtung (1) zur Erfassung von Herzrhythmusstörungen mit
- ein Sensorelement (3), das so konfiguriert ist, dass es ein Herzsignal des Herzens eines Patienten ermittelt, und
- einen Prozessor (2), der so konfiguriert ist, dass er
- Bereitstellen eines Eingangssignals (5), das sich auf das Herzsignal bezieht, wobei das Eingangssignal (5) kardiale Ereignisse (6) und Rauschereignisse (7) umfasst, wobei die kardialen Ereignisse (6) mit der interessierenden Herzaktivität des Herzens des Patienten in Beziehung stehen, und wobei die Rauschereignisse (7) nicht mit der interessierenden Herzaktivität des Herzens in Beziehung stehen;
- Bestimmen von kardialen Ereignissen (6) aus dem Eingangssignal (5) in einem Arrhythmie-Erfassungsfenster (9), und
- Auswerten der kardialen Ereignisse (6) im Arrhythmie-Erfassungsfenster (9) unter Berücksichtigung von mindestens einem Rauschereignis (7), das nach einem Ende des Arrhythmie-Erfassungsfensters (9) auftritt.

## Revendications

1. Méthode de détection d'arythmie au sein du signal cardiaque d'un patient, dans laquelle la méthode est exécutée par un processeur (2) et comprend les étapes consistant à :
- fournir un signal d'entrée (5) qui se réfère au signal cardiaque du patient, dans lequel le signal d'entrée (5) comprend des événements cardiaques (6) et des événements de bruit (7), dans lequel les événements cardiaques (6) sont liés à l'activité cardiaque d'intérêt du cœur du patient, et dans lequel les événements de bruit (7) ne sont pas liés à l'activité cardiaque d'intérêt du cœur ;
- déterminer les événements cardiaques (6) à partir du signal d'entrée (5) dans une fenêtre de détection d'arythmie (9) ;
- évaluer la fenêtre de détection d'arythmie (9) en prenant en compte au moins un événement de bruit (7) se produisant après une fin de la fenêtre de détection d'arythmie (9).

2. Méthode selon la revendication 1, dans laquelle l'étape consistant à déterminer les événements cardiaques (6) à partir du signal d'entrée (5) dans la fenêtre de détection d'arythmie (9) se termine si un événement de bruit (7) est détecté dans la fenêtre de détection d'arythmie (9).

3. Méthode selon la revendication 1 ou 2, dans laquelle l'étape consistant à déterminer les événements cardiaques (6) à partir du signal d'entrée (5) dans la fenêtre de détection d'arythmie (9) se termine si un nombre prédéterminé d'événements cardiaques (6) est détecté dans la fenêtre de détection d'arythmie (9).

4. Méthode selon l'une quelconque des revendications précédentes, dans laquelle la fenêtre de détection d'arythmie (9) est suivie par une période de confirmation (11) après la fin de la fenêtre de détection d'arythmie (9), dans laquelle la fenêtre de détection d'arythmie (9) est confirmée lorsqu'aucun événement de bruit (7) n'est détecté pendant la période de confirmation (11), et dans laquelle la fenêtre de détection d'arythmie (9) est rejetée lorsqu'un événement de bruit (7) est détecté pendant la période de confirmation (11).

5. Méthode selon la revendication 4, dans laquelle la durée de la période de confirmation (11) est déterminée par un nombre prédéterminé d'événements cardiaques (6) après la fin de la fenêtre de détection d'arythmie (9), et dans laquelle le nombre prédéterminé d'événements cardiaques (6) après la fin de la fenêtre de détection d'arythmie (9) dépend du nombre d'événements de bruit (7) se produisant avant le début de la fenêtre de détection d'arythmie (9).

6. Méthode selon l'une quelconque des revendications précédentes, comprenant en outre les étapes consistant à :
- fournir un compteur d'événements (8) qui est configuré pour compter les événements cardiaques (6) et les événements de bruit (7),
- avant le début de la fenêtre de détection d'arythmie (9) :
- augmenter le compteur d'événements (8) d'une première valeur pour chaque événement cardiaque détecté (6) ;
- diminuer le compteur d'événements (8) d'une deuxième valeur pour chaque événement de bruit détecté (7),
- démarrer la fenêtre de détection d'arythmie (9) lorsque le compteur d'événements (8) est supérieur ou égal à une première valeur prédéterminée.

7. Méthode selon la revendication 6, dans laquelle la deuxième valeur est le double de la première valeur.

8. Méthode selon la revendication 6 ou 7, comprenant en outre les étapes consistant à
- après avoir démarré la fenêtre de détection d'arythmie (9),
- augmenter le compteur d'événements (8) de la première valeur pour chaque événement cardiaque détecté (6) ;
- diminuer le compteur d'événements (8) d'une troisième valeur si un événement de bruit (7) est détecté,
- terminer la fenêtre de détection d'arythmie (9) soit
- si le compteur d'événements (8) est inférieur à la première valeur prédéterminée soit
- si le compteur d'événements (8) est supérieur ou égal à une deuxième valeur prédéterminée.

9. Méthode selon la revendication 8, dans laquelle après que la fenêtre de détection d'arythmie (9) se termine parce que le compteur d'événements (8) est supérieur ou égal à la deuxième valeur prédéterminée, une période de terminaison (12) démarre en définissant le compteur d'événements (8) sur une quatrième valeur, dans laquelle pendant la période de terminaison (12), le compteur d'événements (8) diminue avec chaque événement cardiaque (6) qui n'indique pas la présence d'une arythmie, et dans laquelle une séquence de détection d'arythmie se termine dès que le compteur d'événements (8) est supérieur ou égal à la première valeur prédéterminée.

10. Méthode selon l'une quelconque des revendications 1 à 5, comprenant en outre les étapes consistant à :
- fournir un premier compteur d'événements (13) qui est configuré pour compter les événements cardiaques (6),
- fournir un second compteur d'événements (14) qui est configuré pour compter les événements de bruit (7),
- avant le début de la fenêtre de détection d'arythmie (9) :
- augmenter le second compteur d'événements (14) d'une cinquième valeur pour chaque événement de bruit détecté (7) ;
- diminuer le second compteur d'événements (14) d'une sixième valeur pour chaque événement cardiaque détecté (6),
- démarrer la fenêtre de détection d'arythmie (9) lorsque le second compteur d'événements (14) est inférieur ou égal à une troisième valeur prédéterminée,
dans laquelle le premier compteur d'événements (13) démarre lorsque la fenêtre de détection d'arythmie (9) démarre.

11. Méthode selon la revendication 10, comprenant en outre les étapes consistant à
- après avoir démarré la fenêtre de détection d'arythmie (9),
- augmenter le premier compteur d'événements (13) d'une septième valeur pour chaque événement cardiaque détecté (6) ;
- augmenter le second compteur d'événements (14) de la cinquième valeur pour chaque événement de bruit détecté (7),
- terminer la fenêtre de détection d'arythmie (9) soit
- si le premier compteur d'événements (13) est supérieur ou égal à une quatrième valeur prédéterminée soit
- si le second compteur d'événements (14) est supérieur à la troisième valeur prédéterminée.

12. Méthode selon la revendication 11, dans laquelle après que la fenêtre de détection d'arythmie (9) se termine parce que le premier compteur d'événements (13) est supérieur ou égal à la quatrième valeur prédéterminée, une période de confirmation (11) démarre en définissant le second compteur d'événements (14) sur une huitième valeur, et pendant la période de confirmation (11) le second compteur d'événements (14) diminue d'un avec chaque événement cardiaque (6) et un résultat de détection d'arythmie réussi est confirmé dès que le second compteur d'événements (14) est supérieur ou égal à zéro.

13. Méthode selon la revendication 12, comprenant en outre les étapes consistant à
- déterminer une période de terminaison (12), en définissant le premier compteur d'événements (13) sur une neuvième valeur ;
- pendant la période de terminaison (12), diminuer le premier compteur d'événements (13) d'un avec chaque événement cardiaque (6) qui n'indique pas la présence d'une arythmie et terminer la séquence de détection d'arythmie dès que le premier compteur d'événements (13) est inférieur ou égal à la troisième valeur prédéterminée.

14. Dispositif (1) de détection d'arythmie comprenant
- un élément de détection (3) qui est configuré pour déterminer un signal cardiaque du cœur d'un patient, et
- un processeur (2) qui est configuré pour
- fournir un signal d'entrée (5) qui se réfère au signal cardiaque, dans lequel le signal d'entrée (5) comprend des événements cardiaques (6) et des événements de bruit (7), dans lequel les événements cardiaques (6) sont liés à l'activité cardiaque d'intérêt du cœur du patient, et dans lequel les événements de bruit (7) ne sont pas liés à l'activité cardiaque d'intérêt du cœur ;
- déterminer les événements cardiaques (6) à partir du signal d'entrée (5) dans une fenêtre de détection d'arythmie (9), et
- évaluer les événements cardiaques (6) dans la fenêtre de détection d'arythmie (9) en prenant en compte au moins un événement de bruit (7) se produisant après une fin de la fenêtre de détection d'arythmie (9).
